# EUROPEAN PATENT SPECIFICATION

(11) **EP 1 775 344 B1**
(45) Date of publication and mention of the grant of the patent: **10.08.2011**
(21) Application number: 06021211.5
(22) Date of filing: 10.10.2006
(51) Int. Cl.: C12N 9/20, C12N 15/55, C07K 14/40, C12P 7/64, C12P 21/02, C12N 11/00, C12M 1/16

(54) **Lipase, its gene, the strain and the application of this lipase**
Lipase, dessen Gen, der Stamm und Verwendung dieser Lipase
Lipase, son gène, la souche et application de cette lipase

(30) Priority: 11.10.2005 CN 200510112638
(43) Date of publication of application: 18.04.2007
(73) Proprietor: Beijing University of Chemical Technology, Chao Yang District Beijing 100029 (CN)
(72) Inventor: Tan, Tianwei, Beijing 100029 (CN); Yu, Mingrui, Beijing 100029 (CN); Chen, Biqiang, Beijing 100029 (CN); Wang, Fang, Beijing 100029 (CN); Deng, Li, Beijing 100029 (CN); Nie, Kaili, Beijing 100029 (CN); He, Yaoqiang, Beijing 100029 (CN)
(74) Representative: Bachinger-Fuchs, Eva-Maria

(56) References cited:
- WO-A-01/83773
- DATABASE EMBL [Online] 11 May 2000 (2000-05-11), "Yarrowia lipolytica lip2 gene" XP002424703 retrieved from EBI accession no. EMBL:AJ012632 Database accession no. AJ012632 -& PIGNÈDE G ET AL: "Characterization of an Extracellular Lipase Encoded by LIP2 in Yarrowia lipolytica" JOURNAL OF BACTERIOLOGY, WASHINGTON, DC, US, vol. 182, no. 10, May 2000 (2000-05), pages 2802-2810, XP002160655 ISSN: 0021-9193
- BIGEY FRÉDÉRIC ET AL: "Identification of a triacylglycerol lipase gene family in Candida deformans: molecular cloning and functional expression." YEAST (CHICHESTER, ENGLAND) FEB 2003, vol. 20, no. 3, February 2003 (2003-02), pages 233-248, XP002424596 ISSN: 0749-503X
- TAN TIANWEI ET AL: "Screening of high lipase producing Candida sp. and production of lipase by fermentation." PROCESS BIOCHEMISTRY, vol. 39, no. 4, 29 December 2003 (2003-12-29), pages 459-465, XP002424597 ISSN: 1359-5113
- GUIEYSSE D ET AL: "New efficient lipase from Yarrowia lipolytica for the resolution of 2-bromo-arylacetic acid esters" TETRAHEDRON: ASYMMETRY, ELSEVIER SCIENCE PUBLISHERS, AMSTERDAM, NL, vol. 15, no. 22, 15 November 2004 (2004-11-15), pages 3539-3543, XP004636984 ISSN: 0957-4166
- HE XIANG-LING ET AL: "Enzymatic synthesis of 2-ethylhexyl esters of fatty acids by immobilized lipase from Candida sp. 99-125." JOURNAL OF MOLECULAR CATALYSIS B ENZYMATIC, vol. 18, no. 4-6, 23 October 2002 (2002-10-23), pages 333-339, XP002424598 ISSN: 1381-1177
- DENG LI ET AL: "Enzymatic production of fatty acid alkyl esters with a lipase preparation from Candida sp. 99-125." EUROPEAN JOURNAL OF LIPID SCIENCE AND TECHNOLOGY, vol. 105, no. 12, December 2003 (2003-12), pages 727-734, XP002424599 ISSN: 1438-7697
- DENG L ET AL: "Studies on production of biodiesel by esterification of fatty acids by a lipase preparation from Candida sp. 99-125" CHINESE JOURNAL OF CHEMICAL ENGINEERING, CHEMICAL INDUSTRY PRESS, BEIJING, CN, vol. 13, no. 4, August 2005 (2005-08), pages 529-534, XP008076494 ISSN: 1004-9541
- DATABASE BIOSIS [Online] BIOSCIENCES INFORMATION SERVICE, PHILADELPHIA, PA, US; February 2005 (2005-02), NIE KAI-LI ET AL: "Synthesis of biodiesel using immobilized lipase in batch and continous reactors" XP002424704 Database accession no. PREV200510218627 & SHENGWU JIAGONG GUOCHENG, vol. 3, no. 1, February 2005 (2005-02), pages 58-62, ISSN: 1672-3678
- DESTAIN J ET AL: "Lipase from Yarrowia lipolytica" MEDEDELINGEN VAN DE FACULTEIT LANDBOUWWETENSCHAPPEN UNIVERSITEIT GENT, GENT, BE, vol. 62, no. 4A/B, 1997, pages 1223-1229, XP002105079 ISSN: 0368-9697
- DATABASE EMBL [Online] 30 July 2006 (2006-07-30), "Yarrowia lipolytica lipase 2 gene, complete cds." XP002424705 retrieved from EBI accession no. EMBL:DQ831123 Database accession no. DQ831123
- DE OLIVEIRA ET AL: "Influence of compressed fluids treatment on the activity of Yarrowia lipolytica lipase" JOURNAL OF MOLECULAR CATALYSIS. B, ENZYMATIC, ELSEVIER, AMSTERDAM,, NL, vol. 39, no. 1-4, 2 May 2006 (2006-05-02), pages 117-123, XP005389725 ISSN: 1381-1177
- TAN ET AL: "Enzymatic synthesis of 2-ethylhexyl palmitate by lipase immobilized on fabric membranes in the batch reactor" BIOCHEMICAL ENGINEERING JOURNAL, ELSEVIER, AMSTERDAM, NL, vol. 29, no. 1-2, 1 April 2006 (2006-04-01), pages 41-45, XP005335966 ISSN: 1369-703X
- YIN C ET AL: "Synthesis of vitamin A esters by immobilized Candida sp. lipase in organic media" CHINESE JOURNAL OF CHEMICAL ENGINEERING, CHEMICAL INDUSTRY PRESS, BEIJING, CN, vol. 14, no. 1, February 2006 (2006-02), pages 81-86, XP008076426 ISSN: 1004-9541
- ROENNE TORBEN H ET AL: "Lipase-catalyzed esterification of lactic acid with straight-chain alcohols" JOURNAL OF THE AMERICAN OIL CHEMISTS' SOCIETY, vol. 82, no. 12, December 2005 (2005-12), pages 881-885, XP002424635 ISSN: 0003-021X
- YU M ET AL: "Purification and characterization of the extracellular lipase Lip2 from Yarrowia lipolytica" PROCESS BIOCHEMISTRY, vol. 42, 2007, pages 384-391, XP002424600 ISSN: 1359-5113
- TRACEWELL C A ET AL: "Directed enzyme evolution: climbing fitness peaks one amino acid at a time", CURRENT OPINION IN CHEMICAL BIOLOGY, CURRENT BIOLOGY LTD, LONDON, GB LNKD- DOI:10.1016/J.CBPA.2009.01.017, vol. 13, no. 1, 1 February 2009 (2009-02-01), pages 3-9, XP026058907, ISSN: 1367-5931 [retrieved on 2009-02-25]
- HE Y-Q ET AL: "Use of response surface methodology to optimize culture medium for production of lipase with Candida sp. 99-125", JOURNAL OF MOLECULAR CATALYSIS B, vol. 43, 11 July 2006 (2006-07-11), pages 9-14, XP025156545,

## Description

The present invention relates to a lipase having a amino acid sequence as shown SEQ ID:1. The invention also relates to a gene encoding the lipase and an yeast of *Yarrowia lipolytica* to produce the lipase. The invention further relates to methods of using the lipase for production of esters.

### Field

This present invention relates to an isolated yeast lipase. The invention also relates to a polynucleotide encoding this lipase. The invention further relates to the strain employed to produce the lipase. The invention further relates to the methods of using this lipase to synthesize various esters.

### Background

Esters are widely used in food, beverage, medicine and bioenergy. Fatty acid esters are usually synthesized by chemical methods using sulfuric acid as catalyst under high temperature and pressure. Although the yield is high, there are a lot of shortage such as high energy consumption, many by-products, difficult separation and purification. With the development of biotechnology, especially the enzyme engineering, there are many new methods to synthesize fatty acid esters by biocatalysts. Compared with chemical methods, the enzymatic process has many advantages such as low temperature, high selectivity, less by-products and good product quality.

The patent "esters synthesis by microorganism lipase (CN1223300A)" reported a process of fatty acid synthesis by free lipase at 15-40°C using fatty acid and alcohol as substrates. The lipase was produced by *Rhizopus chinensis.* The reaction was a batch-process in a stirred tank. The conversion can reach 95% after 10-36h. Because the free lipase was used, the reusability of the enzyme was not good and the product separation was a little difficult.

Leite et al. reported esters synthesis by immobilized lipase from *Mucor miehei* in isooctane. Bufeiend et al. reported that long chain fatty acid esters were synthesized in hollow-membrane reactor using octane as the solvent. Although the reaction was very fast in the membrane reactor, the cost of the membrane reactor was high. *Yarrowia lipolytica* has been classified as Generally Regarded As Safe (GRAS) by the American Food and Drug Administration (FDA) for citric acid and single cell protein production. *Y. lipolytica* can produce several extracellular proteins including protienases, lipases, RNAases and esters (Barth and Gaillardin, 1997). In 1976, Ota et al. described both an extracellular lipase and two cell-bound lipases in *Y. lipolytica.* Ota et al. purified the 39 kDa extracellular lipase and determined the N-terminal amino acid sequence in 1996. In 1997, Destain et al. reported that *Y.* /*ipolytica* was used for large-scale fermentation to produce lipase. The lipase activity can reach 1000 U/ml. In 2000, Fignede et al. improved the production of the extracellular lipase Lip2. The activity can reach 10000U/ml under the optimum conditions in 15L fermentor. Although the activity is very high, the productin scale is small and it is difficult to scale-up. Sandoval et al. reported the chiral resolution of organic acids using *Y*. *lipolytica* lipase. The ee of products is very high. Tianwei Tan et al.(Process Biochemistry 39 (2003) 459-465) describe a mutant strain Candida sp.99-125 with high lipase activity, which reached 8060 U/ml in a 1500L fermenter.

### Detailed description

To overcome the shortage mentioned above, this invention claims a new lipase, its gene, the strain by which it is obtained and the application of this lipase.

The present invention is directed to a lipase, its amino acid shown in SEQ ID NO: 1. SEQ ID NO:1 consists of 301 amino acids. A conserved pentapeptide (GHSLG) from164 to 168 is conserved in this sequence. Compared with the amino acid sequence encoded by Lip2 gene (Genebank accession no. AJ012632) form *Y. lipolytica,* the 179^{th} amino acid is mutated from G to D

The invention claims the lipase gene, the sequence shown in SEQ ID NO:2 containing 906 bases. Compared with Lip2 gene, the 536^{th} is mutated from g to a. The invention relates to the nucleotide shown in SEQ ID NO:2.

The invention relates to the strain which produces the lipase. The strain was deposited in China General Microbiological Culture Collection Center as CGMCC NO.1470 The invention relates to a process for the enzymatic production of esters using the above lipase The lipase can be obtained by purification from the above *Y*. *lipolytica* strain CGMCC No.1470,

### b) Ester synthesis by immobilized lipase

Immobilized lipase contains lipase, support and co-immobilized agent. The support was mixed with 1-3 fold co-immobilized agent. Then the mixture was air-dried. Lipase was dissolved in distilled water and mixed with co-immobilized agent. The support include gel, Celite and textile including natural textile such as cotton and chemical textile such as terylene. The immobilized lipase with membrane-texitle has a lot of advantage such as low cost, good stability and reusability. Co-immobilized agents include PEG6000, coconut oil, Tween 80, glutin and MgSO4. The concentration is 0.5-2% (W/V). The ratio of these was 5:1:1:2:1:1.

One process of this invention is particularly suitable for the preparation of esters by esterification of fatty acids and alcohols. In a preferred embodiment, the esterification was carried out in a stirred tank reactor by reacting a fatty acid and an alcohol with a molar ratio of 3:1-1:5 in the presence of an immobilized lipase catalyst at 20-50°C. Molecular sieves or silica gels were added into the reactor to remove the water produced by the esterification reaction. The reaction proceeded for 9-50h and a conversion of 56-97% could be obtained. The lipase catalyst was removed by filtration and washed with organic solvents for reuse. The product was obtained after removing the solvent by evaporation under reduced pressure 0.05-0.08Mpa.

The equation of the esterification reaction in the present invention is:

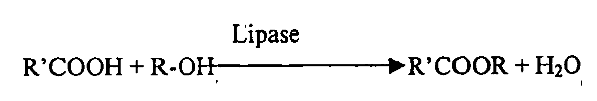

One unit of activity is equivalent to the amount of enzyme required to liberate 1µmol free fatty acids per minute from olive oil at 40°C.

The fatty acid described in the above equation is one of C8-18 mono- or dicarboxylic acids. In a preferred embodiment, the acids caprylic acid, capric acid, palmitic acid, stearic acid, oleic acid, linoleic acid, linolenic acid, adipic acid, azelaic acid and sebacic acid. The alcohol is one of Cl-3 primary alcohols, C3-5 secondary alcohols, glycerol, Vitamin C. In a preferred embodiment, the alcohols are methanol, propanol, butanol, hexanol, isooctanol, isopropanol, isobutanol, decanol, lauryl alcohol, octadecyl alcohol, glycerol and Vitamin C.

For this process, the suitable organic solvents are acetone, hexane, cyclohexane, petroleum ether, heptane and octane. A solvent free system can be also employed for some liquid substrates.

Another process of this invention is suitable for the preparation of esters in procedure (b) by interesterification of fatty acids or alcohols and esters. In a preferred embodiment, the interesterification was carried out in a stirred tank reactor by reacting a fatty acid or an alcohol and an ester with a molar ratio of 3:1-1:7 in the presence of an immobilized lipase catalyst at 20-50°C. Molecular sieves or silica gels were added into the reactor to remove the water produced by the esterification reaction. The reaction proceeded for 9-50h and a conversion of 80-93% could be obtained. The lipase catalyst was removed by filtration and washed with organic solvents for reuse. The product was obtained after removing the solvent by evaporation under reduced pressure 0.05-0.08Mpa.

The equations of the esterification reaction in the present invention are: 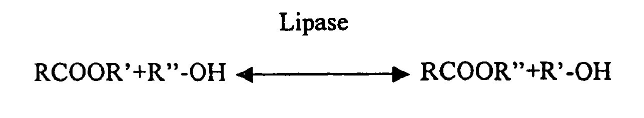 or

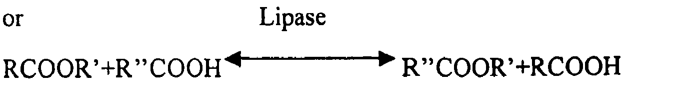

The substrate fatty acid described in the above equations is one of palmitic acid and oleic acid; the substrate alcohol is one of methanol, ethanol; the substrate ester is one of Vitamin A acetate, bean oil, safflower oil, flaxseed oil, corn oil, palm oil and salad oil.

For this process, the suitable organic solvents are acetone, hexane, cyclohexane, petroleum ether, heptane and octane. A solvent free system can be also employed for some liquid substrates.

The advantage of this invention is to obtain a high-producing lipase strain though a series of mutation. High lipase activity (8000U/ml culture broth) was obtained in 500 L fermentor under the optimum conditions. The specific activity can reach 18600 U/mg after purification by ion-exchange chromatography and hydrophobic interaction chromatography. The lipase can use various substrates including acids, alcohols, glycerol, Vitamin A and C. The immobilized lipase shows good stability and reusability.

### Legends of figures

Fig.1 Mutation process I. UV light; n: quick neutron; γ : γ -ray; ME: magnetic field.
Fig.2 Mutation process II. n: quick neutron; γ : γ -ray; ME: magnetic field.
Fig.3 Effect of nitrogen sources on lipase production a: soybean meal; b: defatted soybean meal.
Fig.4 Effect of temperature on lipase production.
Fig.5 Effect of pH on lipase production.
Fig.6 Lipase production in 500L fermentor.

### Example 1 Yarrowia lipolytica

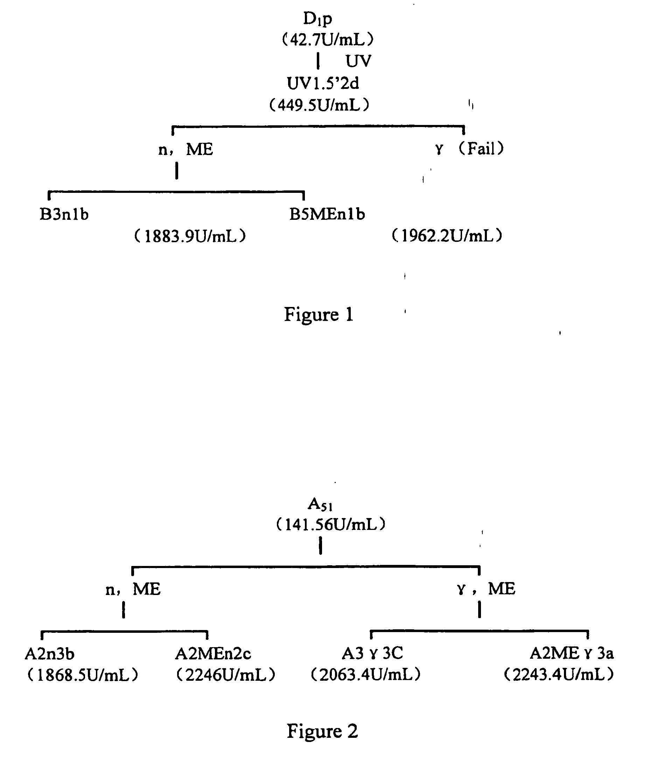

This strain is a high lipase producing strain which is mutated by UV, quick neutron, γ -ray and magnetic field. In brief, the mutation process was shown in Fig. 1 and 2. Finally, by a series of mutation, high lipase producing strain with 2246 U/ml activity was obtained.

### Example 2 Optimization of fermentation

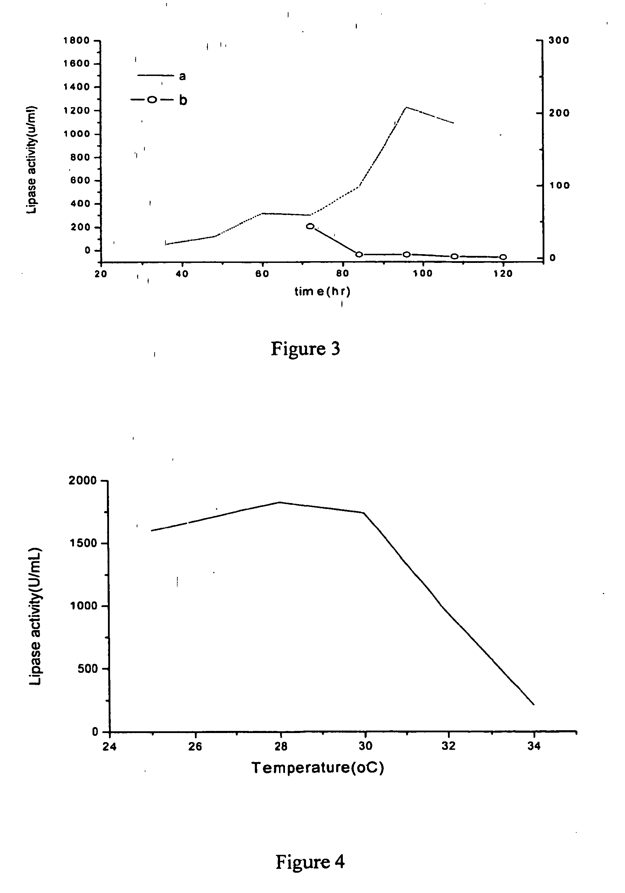

The effect of nitrogen sources on lipase production was investigated. Fig.3 showed that the lipase activity is low when soybean meal was used as nitrogen source.

The effect of carbon sources on lipase production was also investigated. As shown in Table 1, soybean oil can improve the lipase production.

**Table 1**

| time, h | 24 | 48 | 72 | 96 |
|---|---|---|---|---|
| Soybean oil | 440 | 960 | 1520 | 1760 |
| Rapeseed oil | 360 | 720 | 1340 | 1480 |
| Olive oil | 320 | 640 | 880 | 1080 |

The effect of temperature on lipase production was shown in Fig. 4. The optimum temperature for lipase production was 28-30 °C . The initial pH of the medium influenced the lipase production. As shown in Table 2, the optimum pH was in the range of 6.0-7.0.

**Table 2**

| | | | | | | | |
|---|---|---|---|---|---|---|---|
| Initial pH | 4 | 5 | 6 | 7 | 8 | 9 | Natural pH |
| Final pH | 4 | 4.8 | 5.6 | 6.4 | 6.7 | 7.2 | 5.7 |
| activity (U/ml) | 400 | 1460 | 1680 | 1600 | 1040 | 700 | 1620 |

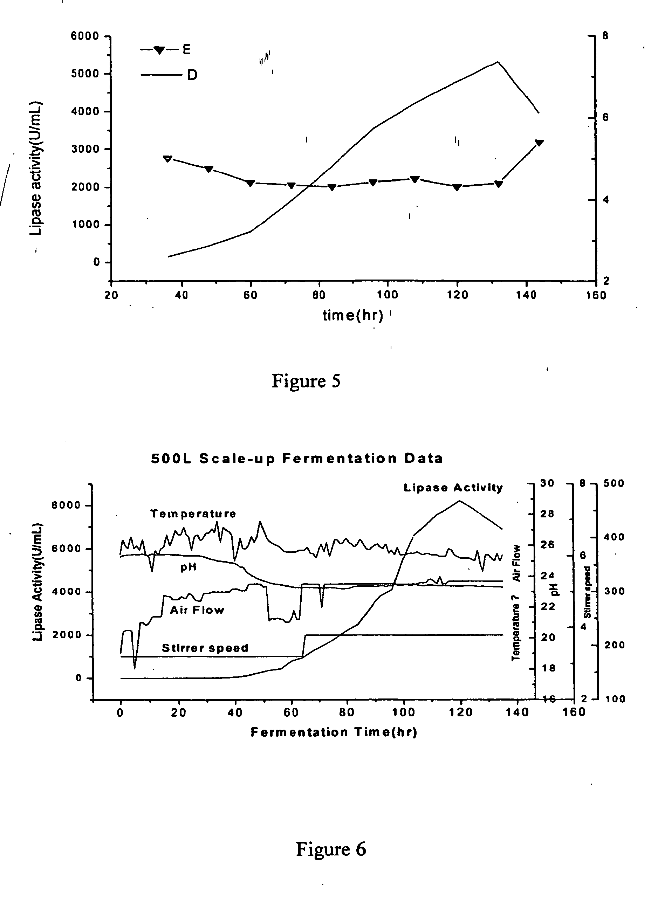

The lipase production was studied in 30L fermentor using optimum culture medium (4% (w/v) defatted soybean meal, 2.5% (w/v) soybean oil, 0.1% (w/v) K2HPO4, 0.1 % (w/v) Span 60). The result was shown in Fig. 5. The lipase activity increased slowly for the first 40 h and then started increasing more strongly and finally reached 5300 U/ml after 130 h.

### Example 3 Fermentation and purification of lipase

The fermentation was scaled up to 500 L fermentor. The medium consisted of 4% (w/v) soybean meal, 2.5% (w/v) soybean oil, 0.1 % (w/v) K2HPO4, 0.1 % (w/v) Span 60, 0.3% antifoam. The highest lipase activity (8000U/ml) was obtained at 26 °C at 400 rpm with 0.35 vvm (Fig.6).

Purification of lipase was carried out with normal purification tehcnology. In brief, the culture broth was centrifuged at 6000 g for 10 min. 4% (w/v) PEG 6000 was added to the supernatant. The mixture was centrifuged at 6000 g for 20 min and the lower layer was added with 3 volume cold acetone. The precipitation was air-dried and crude lipase extract with 5×10⁴ U/g was obtained.

### Example 4 Cloning of lipase gene

The crude lipase extract was dissolved with 25 mM phosphate buffer (pH 7.50). The solution was centrifuged at 10000 g for 10 min at 4 °C. The supernatant was loaded on Q sepharose FF column. Then 1 M NaCl solution was used to elute the lipase component. The lipase solution was pooled together. Ammonium sulfate powder was added to the lipase solution to the concentration of 0.8 M. Then it was loaded on Butyl sepharose FF column. The lipase was eluted with 25 mM Tris-HCl buffer (pH 7.50). SDS-PAGE showed that the purity of the lipase was >90%. The lipase band on SDS-PAGE was transferred to PVDF membrane. The first ten N-terminal amino acids were VYTSTETSHI. The result showed that the N-terminal amino acid matched to the amino acid encoded by LIP2 gene. Pentapeptide (GHSLG) was conserved in the LIP2 sequence. According to LIP2 gene (SeQ NO. 1), the primers were designed as follows:
Primer 1: 5'-ACGAGAATTCGTGTACACCTCTACCGAGACC-3'
Primer 2: 5'-AATAAAGCTTGATACCACAGACACCCTCGGT 3'

The lipase gene was amplified by PCR using the genome DNA of *Yarrowia lipolytica.* The sequence of the gene (SEQ ID NO:2) was homologous to lip2 gene and coding the mature peptides of the lipase. Just the g of the 536 position in nucleotide acids was mutated to a, resulting in the glycin (G) of 179 position was changed to aspartic acid (D) in amino acids sequence.

### Example 5 Immobilization of lipase

The cotton fabric and the co-immobile reagent were mixed according to the proportion of 1:1 (mass:volumn). The activated carrier was obtained after airing at room temperature. The co-immobilized reagents were the mixture of PEG6000, coconut oil, tween 80, glutin, lecithin and magnesium sulphate. The mass proportion of glutin, lecithin, PEG6000, tween 80, magnesium sulphate and coconut oil was 5:1: 1: 2: 1: 1. The lipase solution of *Yarrowia lipolytica* was mixed with activated carrier via 8000 U per gram, marinated and dried at room temperature so as to get the immobilized lipase.

### Example 6

The diatomite and the co-immobilized reagent were mixed according to the proportion of 1:1.5 (mass:volumn). The activated carrier was obtained after airing at room temperature. The co-immobilized reagents were the mixture of PEG6000, coconut oil, tween 80, glutin, lecithin and magnesium sulphate. The mass proportion of glutin, lecithin, PEG6000, tween 80, magnesium sulphate and coconut oil was 5:1: 1: 2: 1: 1. The lipase solution of *Yarrowia lipolytica* was mixed with activated carrier via 8000 U per gram, marinated and dried at room temperature so as to get the immobilized lipase.

### Example 7

The terylene and the co-immobilized reagent were mixed according to the proportion of 1:3 (mass:volumn). The activated carrier was obtained after airing at room temperature. The co-immbile reagents were the mixture of PEG6000, coconut oil, tween 80, glutin, lecithin and magnesium sulphate. The mass proportion of glutin, lecithin, PEG6000, tween 80, magnesium sulphate and coconut oil was 5: 1: 1: 2: 1 1: 1. The lipase solution of *Yarrowia lipolytica* was mixed with activated carrier via 8000IU per gram, marinated and dried at room temperature so as to get the immobilized lipase.

### Example 8

0.085 g immobilized lipase (16000u/g) in example 5 was added into 5 ml hexane which contained 0.85mol/L oleic acid and methanol. After 16 hours at 40°C, the conversion ratio of the reaction was 95%. The mixture was then distilled under reduced pressure (-0.05Mpa) to afford the methane oleic.

### Example 9

0.12 g immobilized lipase (9000u/g) in example 5 was added into 5 ml peroleum ether which contained 1 g hexadecanoic acid (0.54mol/L) and 0.67 g 2-ethtl hexanol (0.7 mol/L). The solution was placed in an orbital shaker at 40°C and 190 r.p.m. After 22 hours the conversion ratio was 96.7%. The half life reached 150 hours.

### Example 10

0.05 g immobilized lipase (16000u/g) in example 5 was added into 5 ml hexamethylene which contained 0.7mol/L stearic acid and butanol. After 24 hours at 40□, the conversion ratio of the reaction was 97%. The solution was then distilled under reduced pressure (-0.05Mpa) to afford the butanol stearic.

### Example 11

0.12 g immobilized lipase (9000u/g) in example 5 was added into 5 ml heptane which contained 1 g stearic acid and 0.62 g 2-ethtl hexanol (0. 65 mol/L). The solution was placed in an orbital shaker at 45°C and 190 r.p.m. After 50 hours the conversion ratio was 95%.

### Example 12

20 g immobilized lipase (9000u/g) in example 5 was added into 690 ml Peroleum ether which contained 138 g hexadecanoic acid (0.54mol/L) and 92 g 2-ethtl hexanol (0.706 mol/L). The solution was stirred at 40°C. Lipase was immobilized on stainless steel wire net and placed in the reactor. After 24 hours the conversion ratio was 95%. The reaction mixture was lyed , filtered and distilled at vacuum (0.05-0.08Mpa) to afford isooctyl palmitate. The product color was light and the acid number was 0.205mgKOH/g

### Example 13

0.2 g immobilized lipase (9000u/g) in example 5 was added into 20 ml hexane which contained 0.2 g decanoate and 0.147 g isobutanol. The mixture was placed in an orbital shaker at 50°C and 190 r.p.m. After 25 hours the conversion ratio was 91%.

### Example 14

0. 2 g immobilized lipase (9000u/g) in example 6 was added into 20 ml hexane which contained 0.2 g octanoic acid and 0.135 g isopropanol. The mixture was placed in an orbital shaker at 35°C and 190 r.p.m. After 20 hours the conversion ratio was 93%.

### Example 15

0. 3 g immobilized lipase (9000u/g) in example 5 was added into 10 ml hexane which contained 0.2 g decanedioic acid and 0.147 g isobutanol. The mixture was placed in an orbital shaker at 40°C and 190 r.p.m. After 9 hours the conversion ratio was 91%.

### Example 16

0. 12 g immobilized lipase in example 5 was added into 20 ml peroleum ether which contained 0.2 g decanedioic acid and 0.535 g octadecanol. The mixture was placed in an orbital shaker at 30°C and 190 r.p.m. After 12 hours the conversion ratio was 89%.

### Example 17

0. 3 g immobilized lipase in example 7 was added into 10 ml hexane which contained 0.2 g decanedioic acid and 0.2575 g octanol. The mixture was placed in an orbital shaker at 50°C and 190 r.p.m. After 18 hours the conversion ratio was 88%.

### Example 18

0. 25 g immobilized lipase in example 6 was added into 20 ml octane which contaihed 0.2 g hexanedioic acid and 0.3886 g lanryl alcohol. The mixture was placed in an orbital shaker at 20°C and 190 r.p.m. After 48 hours the conversion ratio was 89%.

### Example19

0. 3 g immobilized lipase was added into 20 ml hexane which contained 0.2 g hexanedioic acid and 0.203 g heptanol. The mixture was placed in an orbital shaker at 37°C and 190 r.p.m. After 24 hours the conversion ratio was 92%.

### Example 20

0. 2 g immobilized lipase in example 5 was added into 0.2 g decanedioic acid and 2 g hexanol without solvent. The mixture was placed in an orbital shaker at 40°C and 190 r.p.m. After 20 hours the conversion ratio was 94%.

### Example 21

0. 16 g immobilized lipase in example 5 was added into 0.2 g hexanedioic acid and 2 g octanol without solvent. The mixture was placed in an orbital shaker at 35°C and 190 r.p.m. After 50 hours the conversion ratio was 90%.

### Example 22

0.50 g immobilized lipase in example 5 was added into 10 ml acetone which contained 0.0956 g vitamin C and 1.051 g hexadecanoic acid. After 72 hours at 40°C, the conversion ratio of the reaction was 65.3%.

### Example 23

0.60 g immobilized lipase in example 7 was added into 10 ml acetone which contained 0.0956 g vitamin C and 0.956 g lauric acid. After 72 hours at 37°C, the conversion ratio of the reaction was 56.5%.

### Example 24

0.40 g immobilized lipase was added into 10 ml acetone which contained 0.0956 g vitamin C and 1.203 g tetradecanoic acid. After 72 hours at 40°C, the conversion ratio of the reaction was 58.2%.

### Example 25

0.50 g immobilized lipase in example 5 was added into 10 ml acetone which contained 0.0956 g vitamin C and 1.000 g linoleic acid. After 72 hours at 35°C, the conversion ratio of the reaction was 61.6%.

### Example 26

0.65 g immobilized lipase in example 5 was added into 10 ml acetone which contained 0.0956 g vitamin C and 1.203 g flax acid. After 72 hours at 40°C, the conversion ratio of the reaction was 63.6%.

### Example 27

0.3 g immobilized lipase in example 5 was added into 10 ml hexane which contained 0.100 g vitamin A acetic ester (0.3mmol) and 0.234 g hexadecanoic acid (0.9mmol). After 42 hours at 35°C, the conversion ratio of the reaction was 84%.

### Example 28

0.4 g immobilized lipase in example 5 was added into 20 ml hexane which contained 0.100 g vitamin A acetic ester (0.3mmol) and 0.254 g oleic acid (0.9mmol). After 24 hours at 40°C, the conversion ratio of the reaction was 80%.

### Example 29

0.3 g immobilized lipase in example 5 was added into 10 ml peroleum ether which contained 2.00 g salad oil and 0.25 g methanol. After 30 hours at 40°C, the conversion ratio of the reaction was 93%.

### Example 30

0.3 g immobilized lipase in example 5 was added into 10 ml heptane which contained 2.00 g bean oil and 0.27 g methanol. After 40 hours at 35°C, the conversion ratio of the reaction was 92%.

### Example 31

0.4 g immobilized lipase in example 5 was added into 10 ml peroleum ether which contained 2.00 g safflower oil and 0.30 g methanol. After 50 hours at 40°C, the conversion ratio of the reaction was 93%.

### Example 32

0.35 g immobilized lipase in example 5 was added into 10 ml hexane which contained 2.00 g flax oil and 0.28 g methanol. After 50 hours at 30°C, the conversion ratio of the reaction was 90%.

### Example 33

0.3 g immobilized lipase in example 5 was added into 10 ml peroleum ether which contained 2.00 g corn oil and 0.26 g methanol. After 30 hours at 40°C, the conversion ratio of the reaction was 92%.

### Example 34

0.3 g immobilized lipase in example 6 was added into 10 ml hexamethylene which contained 2.00 g palm oil and 0.29 g methanol. After 40 hours at 30°C, the conversion ratio of the reaction was 89%.

### Example 35

0.3 g immobilized lipase in example 5 was added into 10 ml peroleum ether which contained 2.00 g salad oil and 0.36 g ethanol. After 30 hours at 40°C, the conversion ratio of the reaction was 91%.

### Example 36

0.4 g immobilized lipase in example 5 was added into 20 ml hexane which contained 0.110 g glycerol and 0.190 g decanoate. After 24 hours at 37°C, the conversion ratio of the reaction was 96%.

### Example 37

0.4 g immobilized lipase in example 5 was added into 20 ml hexane which contained 0.110 g glycerol and 0.178 g octanoic acid. After 24 hours at 40°C, the conversion ratio of the reaction was 92%.

SEQ ID: 2
DNA.txt
gtgtacacctctaccgagacctctcacattgaccaggagtcctacaacttctttgagaagtacgcccgactcgcaaacat
tggatattgtgttggtcccggcactaagatcttcaagcccttcaactgtggcctgcaatgtgcccacttccccaacgttg
agctcatcgaggagttccacgacccccgtctcatctttgatgtttctggttacctcgctgttgatcatgcctccaagcag
atctaccttgttattcgaggaacccactctctggaggacgtcataaccgacatccgaatcatgcaggctcctctgacgaa
ctttgatcttgctgctaacatctcttctactgctacttgtgatgactgtcttgtccacaatggcttcatccagtcctaca
acaacacctacaatcagatcggccccaagctcgactctgtgattgagcagtatcccgactaccagattgctgtcaccggt
cactctctcggaggagctgcagcccttctgttcggaatcaacctcaaggttaacgaccacgatcccctcgttgttactct
tggtcagcccattgtcggtaacgctggctttgctaactgggtcgataaactcttctttggccaggagaaccccgatgtct
ccaaggtgtccaaagaccgaaagctctaccgaatcacccaccgaggagatatcgtccctcaagtgcccttctgggacggt
taccagcactgctctggtgaggtctttattgactggcccctgatccaccctcctctctccaacgttgtcatgtgccaggg
ccagagcaataaacagtgctctgccggtaacactctgctccagcaggtcaatgtgattggaaaccatctgcagtacttcg
tcaccgagggtgtctgtggtatc

SEQ ID: 1
protein.txt
VYTSTETSHIDQESYNFFEKYARLANIGYCVGPGTKIFKPFNCGLQCAHFPNVELIEEFHDPRLIFDVSGYLAVDHASKQ
IYLVIRGTHSLEDVITDIRIMQAPLTNFDLAANISSTATCDDCLVHNGFIQSYNNTYNQIGPKLDSVIEQYPDYQIAVTG
HSLGGAAALLFGINLKVNDHDPLVVTLGQPIVGNAGFANWVDKLFFGQENPDVSKVSKDRKLYRITHRGDIVPQVPFWDG
YQHCSGEVFIDWPLIHPPLSNVVMCQGQSNKQCSAGNTLLQQVNVIGNHLQYFVTEGVCGI

## Claims

1. *Yarrowia lipolytica* strain CGMCC No. 1470.

2. A lipase obtained from the yeast strain according to claim 1, having amino acid sequence SEQ ID NO:1.

3. A gene encoding the lipase according to claim 2, having nucleic acid sequence SEQ ID NO:2.

4. A process for the enzymatic production of esters, wherein the lipase according to claim 2 is used.

5. The process according to claim 4, wherein the lipase being used is immobilized.

6. The process according to claim 4 or 5, wherein the esters are produced by esterification of fatty acids and alcohols.

7. The process according to claim 6, wherein the fatty acid is selected from the group consisting of caprylic, capric, palmitic, stearic, oleic, linoleic, linolenic, adipic, azelaic and sebacic acid and the alcohol is selected from the group consisting of methanol, propanol, butanol, hexanol, isooctanol, isopropanol, isobutanol, decanol, lauryl alcohol, octadecyl alcohol, glycerol and Vitamin C.

8. The process according to claim 4 or 5, wherein the esters are produced by transesterificaton of esters and alcohols or acids.

9. The process according to claim 8, wherein the substrate acid is selected from palmitic acid and oleic acid, the substrate ester is selected from the group consisting of Vitamin A acetate, bean oil, safflower oil, flaxseed oil, corn oil, palm oil and salad oil, and the substrate alcohol is selected from methanol and ethanol.

## Patentansprüche

1. *Yarrowia lipolytica -* Stamm CGMCC Nr. 1470.

2. Lipase, welche aus dem Hefestamm nach Anspruch 1 gewonnen wird und die Aminosäuresequenz SEQ ID NO:1 besitzt.

3. Gen, welches die Lipase nach Anspruch 2 kodiert und die Nukleinsäuresequenz SEQ ID NO:2 besitzt.

4. Verfahren zur enzymatischen Herstellung von Estern, wobei die Lipase nach Anspruch 2 verwendet wird.

5. Verfahren nach Anspruch 4, wobei die verwendete Lipase immobilisiert ist.

6. Verfahren nach Anspruch 4 oder 5, wobei die Ester durch Veresterung von Fettsäuren und Alkoholen hergestellt werden.

7. Verfahren nach Anspruch 6, wobei die Festtsäure aus der Gruppe, bestehend aus Capryl-, Caprin-, Palmitin-, Stearin-, Öl-, Linol-, Linolen-, Adipin-, Azelain- und Sebacinsäure, ausgewählt ist und der Alkohol aus der Gruppe, bestehend aus Methanol, Propanol, Butanol, Hexanol, Isooctanol, Isopropanol, Isobutanol, Decanol, Laurylalkohol, Octadecylalkohol, Glycerin und Vitamin C, ausgewählt ist.

8. Verfahren nach Anspruch 4 oder 5, wobei die Ester durch Umesterung von Estern und Alkoholen oder Säuren hergestellt werden.

9. Verfahren nach Anspruch 8, wobei die Substratsäure aus Palmitinsäure und Ölsäure ausgewählt ist, der Substratester aus der Gruppe, bestehend aus Vitamin A-acetat, Sojabohnenöl, Färberdistelöl, Flachssamenöl, Maiskeimöl, Palmöl und Tafelöl, ausgewählt ist und der Substratalkohol aus Methanol und Ethanol ausgewählt ist.

## Revendications

1. Souche de *Yarrowia lipolytica* No. CGMCC 1470.

2. Lipase obtenue à partir de la souche de levure selon la revendication 1, ayant la séquence d'acides aminés SEQ ID NO: 1.

3. Gène codant pour la lipase selon la revendication 2, ayant la séquence d'acide nucléique SEQ ID NO: 2.

4. Procédé pour la production enzymatique d'esters, dans lequel la lipase selon la revendication 2 est utilisée.

5. Procédé selon la revendication 4, dans lequel la lipase utilisée est immobilisée.

6. Procédé selon la revendication 4 ou 5, dans lequel les esters sont produits par estérification d'acides gras et d'alcools.

7. Procédé selon la revendication 6, dans lequel l'acide gras est choisi dans le groupe constitué par l'acide caprylique, caprique, palmitique, stéarique, oléique, linoléique, linolénique, adipique, azélaïque et sébacique et l'alcool est choisi dans le groupe constitué par le méthanol, le propanol, le butanol, l'hexanol, l'isooctanol, l'isopropanol, l'isobutanol, le décanol, l'alcool laurique, l'alcool octadécylique, le glycérol et la vitamine C.

8. Procédé selon la revendication 4 ou 5, dans lequel les esters sont produits par transestérification d'esters et d'alcools ou d'acides.

9. Procédé selon la revendication 8, dans lequel l'acide substrat est choisi parmi l'acide palmitique et l'acide oléique, l'ester substrat est choisi dans le groupe constitué par l'acétate de vitamine A, une huile de graines, l'huile de carthame, l'huile de lin, l'huile de maïs, l'huile de palme et une huile à salade, et l'alcool substrat est choisi parmi le méthanol et l'éthanol.
